# EUROPEAN PATENT APPLICATION

(11) **EP 3 872 813 A1**
(43) Date of publication of application: **01.09.2021**
(21) Application number: 20159531.1
(22) Date of filing: 26.02.2020
(51) Int. Cl.: G16H 10/40

(54) **METHOD FOR ANONYMOUS INFORMATION LOGISTICS**

(71) Applicant: Dynamic Code AB, 582 16 Linköping (SE)
(72) Inventor: Kihlgren, Anne, 582 16 Linköping (SE)
(74) Representative: AWA Sweden AB

(57) **Abstract**

A method (100) for anonymous information logistics, comprising the following steps: providing an article (A); assigning (101) a specific article number (AN) to said article (A); providing a number (n) of said article (A) having a specific article number (AN), thereby forming a lot (L) of articles within said article number; assigning (103) said lot (L) with a single lot number (LN); randomizing (104) individual analysis codes (AC) within said lot number, and providing (105) said individual analysis codes (AC) to said article (A).

## Description

### Technical field

The present document relates to a method for connecting the consumer/patient to the lab and health care provider to providing anonymous identification of personalized test results from medical diagnostic test kits based on self-sampling, laboratory analysis and laboratory test results, provided by pharmacies, hospitals, medical professionals or through internet home sales.

### Background

There are a number of self-test kits available on the market today. Some are of the type where a response is instantly given to the user, within seconds or minutes. One such example is for instance the SELFCheck® tests, where a user can buy a test at a pharmacy or other retailers, and check for gluten sensitivity, blood glucose etc. Other examples include pregnancy tests, which also provide the user with an instant result.

Other types diagnostic tests require that the user sends a sample, such as a blood, tissue or stool sample, or a DNA sample, to an analyzing facility, such as a hospital laboratory or a private laboratory, for instance for a DNA analysis, or other types of analysis. These types of diagnostics tests may, for instance, be bought at a pharmacy, or similar retailer, in a web shop, or be provided by the users' medical contacts, such as a primary care facility, or through a web based medical care. These types of diagnostic tests are for instance provided by Dynamic Code.

If the analysis is performed at a hospital, the patient usually needs to state his or hers personal details, and the secrecy rules of the hospital are intended to safeguard an individuals privacy, even though this secrecy can quite easily be compromised or breached due to human errors.

When such a diagnostic sample is sent to public or private health care facilities for instance a hospital, laboratory or primary health care provider, there is definite need for an information and communication method and platform which allows the user to send in, and retrieve his or hers test result, completely anonymously. This is particularly important when considering biological samples as for example blood, semen, buccal samples that includes DNA from a person.

An information and communication method and platform allowing for complete privacy and anonymity, is needed -to handle tests results in public and private health care facilities.

There is also a need for a method and platform allowing for anonymous handling of diagnostic tests and their test results that consumers can buy and test by themselves. Test that can be distributed by, for example, pharmacies and performed by customers and the diagnosis and health care are given by public or private practices. The test results can be handled anonymously between the lab and the healthcare provider.

### Summary

It is an object of the present disclosure, to provide an improved information and communication method logic, which eliminates or alleviates at least some of the disadvantages of the prior art methods.

According to a first aspect, there is provided a method for anonymous information logistics, comprising the following steps:
a) providing an article;
b) assigning a specific article number to said article;
   providing a number of said article having a specific article number, thereby forming a lot of articles within said article number;
c) assigning said lot with a single lot number;
d) randomizing individual analysis codes within said lot number, and
e) providing said individual analysis codes to said article.
This method provides a way of giving each article a specific analysis code, which can be used to completely anonymously to obtain a test result. Since each article is also assigned an article number, and a lot number there is also full traceability for each individual article, which enables for safety and quality control. Furthermore, each distributor, e.g. pharmacies and healthcare providers, is given a unique customer code. Since, the customer code is coupled to the analysis code it is possible to trace all articles and analysis results, belonging to a certain distributor, and share them to a specific healthcare provider.

According to the first aspect steps b), c), d) are computer implemented. This means that the article numbers, lot numbers, analysis codes and customer code may be generated by a computer, and subsequently stored in a database.

The article may be a diagnostic kit, which may comprise a package and a sampling device.

The diagnostic kit or test may for instance be related to bacterial vaginosis, lactose intolerance, gluten intolerance, urinary tract infections, fungus infections, Strep A infection, etc. The sampling device may be for instance a swab for collecting DNA specimens, or a test tube for collecting fluids or tissue, or a combination thereof.

According to the first aspect in step e) of providing said analysis codes to said articles said analysis code may be printed onto a leaflet, which is placed on or with said article.

This means that the analysis code may for instance be printed onto a leaflet, which is randomly placed in the package of the article within a certain lot. The leaflet may further comprise a sticker provided with said analysis code.

This allows for the user to label the article before sending it to the analysis facility. The leaflet is further provided with a printed analysis code, which the user keeps, to be able to retrieve the test result for instance at web page provided by the analysis facility. The analysis code may also be provided as a combination of letters and number, as a QR code or a bar code or a combination thereof, or any other computer or smart phone readable marking.

According to the first aspect the article number, lot number and analysis codes, and customer code are stored in a database.

This means that there is complete traceability for each article.

The method according to the first aspect may further comprise the steps of:
f) providing an article to a user;
g) labelling said article with the analysis code provided;
h) submitting said labelled article to an analysis facility;
i) analyzing said article, by said analysis facility, thereby providing a test result for said user;
j) retrieving said test result by using the individual analysis code assigned to said article.
k) Combining a distributor to a specific health care provider with full traceability though a unique analysis code

The article may be submitted by the user him/herself or by a laboratory. The article, i.e. the diagnostic test may be used privately (such as at the users home environment) or alternatively, the user may take the test at for instance a primary or community health center, or at a hospital laboratory, label the sample/article with the analysis code and submit it to an analysis facility. The user and/or laboratory thus uses the analysis code for identification of the test result, and there is no need for a user or individual to provide any personalized information which may compromise the anonymity of the individual user.

### Brief Description of the Drawings

Embodiments of the present solution will now be described, by way of example, with reference to the accompanying schematic drawings.
Fig. 1 is a schematic block diagram of an information method according to the present invention.
Fig. 2 is a block diagram of one embodiment of an information method according to the present invention.

### Description of Embodiments

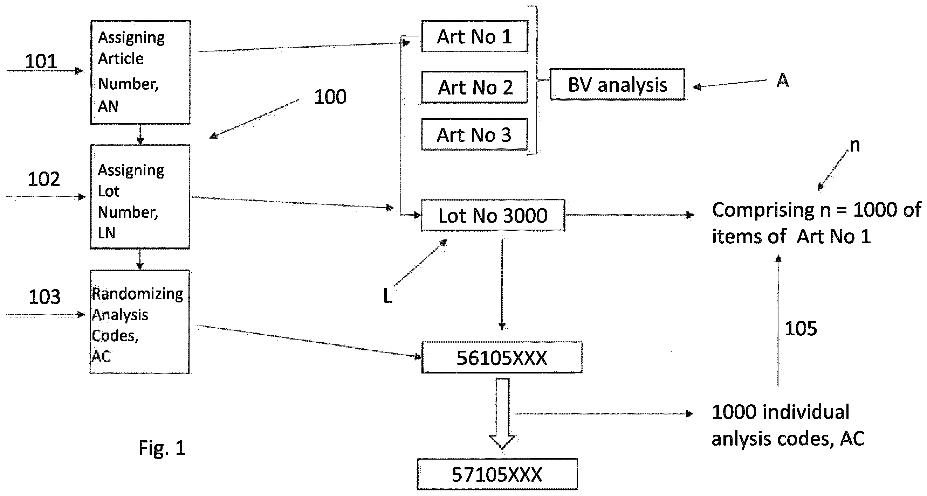

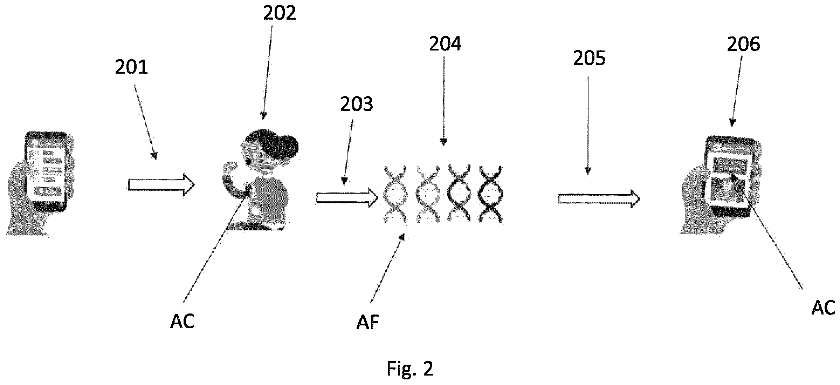

Fig 1 illustrate schematically a method 100 for assigning 101 an article A, in this case exemplified with a diagnostic test for Bacterial Vaginosis analys with an article number AN. One laboratory analysis for instance Chlamydia test may thus be provided with different article number depending on the market (e.g. Sweden or Denmark), and depending on mode of sales (e.g. through pharmacy, online or through a health care provider). As seen in Fig 1 the A BV analysis may be assigned with Art No 1, Art No 2 and Art No 3 etc. These articles often comprise a packaging and a sampling device (as schematically shown in Fig 2).

In the next step the article within one article number AN are assigned 102 one lot number LN, making up a lot L. That means that a (one) lot always comprises one and the same article A. In Fig 1 this is illustrated as Lot NO 3000 comprising 1000 articles of Art No 1, i.e. 1000 BV analysis tests. Following the assignment of a lot number, analysis codes AC are randomized 103 within the lot number LN, i.e. 1000 different analysis codes are outputted, in Fig 1 this is illustrated by 56105XXX → 57105XXX.

A control mechanism is provided in the information system to prevent that the same analysis code is used twice, i.e. the analysis codes are always unique numbers.

These analysis codes may, for instance be printed into the article, or onto a leaflet or other type of information means and placed onto or into the packaging of the article. In one embodiment the analysis code is also printed onto a sticker, which can be removed and placed in the sampling device, or a test tube when a user has taken a sample.

Fig 2 illustrates that a user may order an article A online, but the article may also be purchased at a pharmacy or be handed out by a laboratory or a health care provider. The article A is provided 201 to a user, which obtains the specific sample for the specific diagnostic test. The user then labels 202 the article A with the individual analysis code provided in connection with the article. The user sends 203 the article A to an analysis facility, that performs the analysis 204, and provides 205 the test result to the user, which may retrieve 206 the result online or through a health care provider, by using the individual analysis code. This means that the user is completely anonymous throughout the entire process. The analys facility may be a private facility, or for instance a hospital laboratory.

According to one alternative embodiment, the article number assigned to the article is specific for a certain pharmacy, which may in turn cooperate with a specific health care provider, such as internet-based health care providers. This means that after the step 206 of obtaining the test result the user may be provided with a choice of contacting the health care provider which cooperates with the pharmacy where the diagnostic kit was bought. The user may also use the test result to contact any health care provider for relevant care and medications.

In one embodiment, the test result automatically may result in a prescription being available to the user.

## Claims

1. A method (100) for anonymous information logistics, comprising the following steps:
I) providing an article (A);
m) assigning (101) a specific article number (AN) to said article (A); providing a number (n) of said article (A) having a specific article number (AN), thereby forming a lot (L) of articles within said article number;
n) assigning (103) said lot (L) with a single lot number (LN);
o) randomizing (104) individual analysis codes (AC) within said lot number, and
p) providing (105) said individual analysis codes (AC) to said article (A).

2. The method of claim 1, wherein steps b), c), d) are computer implemented.

3. The method of claim 1 or 2, wherein said article (N) is a diagnostic kit.

4. The method of claim 3, wherein said diagnostic kit comprises a package and a sampling device.

5. The method as claimed in any one of the preceding claims, wherein in the step e) of providing said analysis codes to said articles said analysis code is printed onto a leaflet, which is placed on or with said article.

6. The method as claimed in claim 4, wherein said leaflet comprises a sticker provided with said analysis code (AC).

7. The method as claimed in claim 1, wherein said, article number (AN), lot number (LN) and analysis codes (AC) are stored in a database.

8. The method as claimed in any of the preceding claims, wherein the method further comprises the steps of:
q) providing (201) an article (A) to a user;
r) labelling (202) said article (A) with the analysis code (AC) provided;
s) submitting (203) said labelled article (A) to an analysis facility (AF);
t) analyzing (204) said article (A), by said analysis facility, thereby providing (205) a test result for said user;
u) retrieving (206) said test result by using the individual analysis code (AC) assigned to said article.

## Amended claims

### Amended claims in accordance with Rule 137(2) EPC.

1. A method (100) for anonymous information logistics, comprising the following steps:
a) providing an article (A);
b) assigning (101) a specific article number (AN) to said article (A); providing a number (n) of said article (A) having a specific article number (AN), thereby forming a lot (L) of articles within said article number;
c) assigning (103) said lot (L) with a single lot number (LN);
d) randomizing (104) individual analysis codes (AC) within said lot number, and
e) providing (105) said individual analysis codes (AC) to said article (A),
wherein steps b), c), d) are computer implemented
**characterized in that**
said article number (AN), lot number (LN) and analysis codes (AC) are stored in a database.

2. The method of claim 1, wherein said article (N) is a diagnostic kit.

3. The method of claim 2, wherein said diagnostic kit comprises a package and a sampling device.

4. The method as claimed in any one of the preceding claims, wherein in the step e) of providing said analysis codes to said articles said analysis code is printed onto a leaflet, which is placed on or with said article.

5. The method as claimed in claim 4, wherein said leaflet comprises a sticker provided with said analysis code (AC).

6. The method as claimed in any of the preceding claims, wherein the method further comprises the steps of:
f) providing (201) an article (A) to a user;
g) labelling (202) said article (A) with the analysis code (AC) provided;
h) submitting (203) said labelled article (A) to an analysis facility (AF);
i) analyzing (204) said article (A), by said analysis facility, thereby providing (205) a test result for said user;
j) retrieving (206) said test result by using the individual analysis code (AC) assigned to said article.
